# EUROPEAN PATENT SPECIFICATION

(11) **EP 4 561 504 B1**
(45) Date of publication and mention of the grant of the patent: **26.08.2026**
(21) Application number: 23751723.0
(22) Date of filing: 21.07.2023
(51) Int. Cl.: A61F 2/44

(54) **INTERBODY IMPLANT HAVING ENDPLATES CONNECTED BY STRUTS**
ZWISCHENWIRBELIMPLANTAT MIT DURCH STREBEN VERBUNDENEN ENDPLATTEN
IMPLANT INTERSOMATIQUE AYANT DES PLAQUES D'EXTRÉMITÉ RELIÉES PAR DES ENTRETOISES

(30) Priority: 29.07.2022 US 202217876635
(43) Date of publication of application: 04.06.2025
(73) Proprietor: Warsaw Orthopedic, Inc., Warsaw, IN 46582 (US)
(72) Inventor: MILLER, Keith, Memphis, Tennessee 38132 (US); WOLFE, Thomas, Vestavia Hills, Alabama 35216 (US); METCALF, Newton, Memphis, Tennessee 38132 (US); BALLARD, Rodney, Memphis, Tennessee 38132 (US); PREVOST, Julien, Memphis, Tennessee 38132 (US); BEALE, Jeffrey, Memphis, Tennessee 38132 (US)
(74) Representative: Viering, Jentschura & Partner mbB Patent- und Rechtsanwälte
(86) International application number: PCT/IB2023/057449
(87) International publication number: WO 2024/023671

(56) References cited:
- WO-A2-2004/016205
- WO-A2-2004/016217
- US-A1- 2004 111 155
- US-A1- 2007 050 032
- US-A1- 2010 016 970
- US-A1- 2019 274 838
- US-B1- 9 060 876

## Description

This application makes reference to the following applications: U.S. Pat. App. No. 17/320,441 titled Spinal Implant System and Method, and filed on May 14, 2021; U.S. Pat. No. 11,096,796, titled Interbody spinal implant having a roughened surface topography on one or more internal surfaces, and filed on March 4, 2013; and U.S. Pat. No. 10,821,000, titled Titanium implant surfaces free from alpha case and with enhanced osteoinduction, and filed June 29, 2017.

### FIELD

In a first aspect, the present technology is generally related to surgical implants and devices for insertion in the human body, e.g., between adjacent vertebrae of the human spine. In a second aspect, the present technology is generally related to methods of manufacture and methods of use (not claimed) of surgical implants and devices for insertion in the human body.

### BACKGROUND

Spinal pathologies and disorders such as degenerative disc disease, disc herniation, osteoporosis, spondylolisthesis, stenosis, rumor, scoliosis and other curvature abnormalities, kyphosis and fracture may result from factors including trauma, disease and degenerative conditions caused by injury and aging. Spinal disorders typically result in symptoms including deformity, pain, nerve damage, and partial or complete loss of mobility.

Non-surgical treatments, such as medication, rehabilitation and exercise can be effective, however, may fail to relieve the symptoms associated with these disorders. Surgical treatment of these spinal disorders includes fusion, fixation, correction, partial or complete discectomy, corpectomy and laminectomy, and implantable prosthetics. As part of these surgical treatments, spinal constructs, such as, for example, bone fasteners, spinal rods and interbody devices can be used to provide stability to a treated region. For example, during surgical treatment, interbody implants can be delivered to a surgical site for fixation with bone to immobilize a join US 2007/050032 and US 2010/016970 A1 disclose examples of composite interbody implants comprising a metallic frame that include upper and lower endplates interconnected by compressible components, such as springs, and further comprising a polymeric compressible core body between said endplates. This disclosure describes an improvement over these technologies.

### SUMMARY

The techniques of this disclosure generally relate to interbody implants formed of a metallic frame component or components and a polymeric body component or components. A metallic frame component includes at least one strut interconnecting a superior endplate and an inferior endplate. In various embodiments, "interconnected" and "interconnecting" may refer to a monolithic or unitary component in which a superior and inferior endplates are "connected" and in other embodiments, "interconnected" and "interconnecting" may refer to a multi-piece frame or component in which a superior and inferior endplate are "connected." A polymeric body component is formed to the metallic frame by an overmold process.

The present invention relates to an implant device according to claim 1 or 9. Preferred embodiments of the invention are set forth in the dependent claims. The present disclosure provides for an interbody implant. The interbody implant includes a metallic frame including an interconnected superior endplate and inferior endplate, and the metallic frame has a first compressive stiffness, for example. The interbody implant further includes a polymeric body formed to the metallic frame by an overmold process, and the polymeric body has a second compressive stiffness, for example. The first compressive stiffness of the metallic frame is about 20% to about 80% of the second stiffness of the body.

In another aspect, the disclosure provides for an interbody implant formed at least partially by an overmold process. In various embodiments, the interbody implant may include a metallic frame having a superior endplate and an inferior endplate interconnected by a plurality of translating struts, and the metallic frame may have a first compressive stiffness, for example. The interbody implant includes a polymeric body formed to the metallic frame by an overmold process, and the polymeric body may have a second compressive stiffness, for example. The first compressive stiffness of the metallic frame may be about 20% to about 80% of the second stiffness of the body.

The details of one or more aspects of the disclosure are set forth in the accompanying drawings and the description below. Other features, objects, and advantages of the techniques described in this disclosure will be apparent from the description and drawings, and from the claims.

### BRIEF DESCRIPTION OF DRAWINGS

FIG. 1 is a front perspective view of an interbody implant.
FIG. 2 is a rear perspective view of an interbody implant.
FIG. 3 is a top-down view of an interbody implant.
FIG. 4 is an exploded parts view of an interbody implant.
FIG. 5 is a perspective view of a first example support system and frame for use with disclosed implant embodiments.
FIG. 6 is a perspective view of a polymer body for use with disclosed implant embodiments.
FIG. 7 is a perspective view of a second example support system and frame for use with disclosed implant embodiments.
FIG. 8 is a perspective view of a third example support system and frame for use with disclosed implant embodiments.
FIG. 9 is a perspective view of a fourth example support system and frame for use with disclosed implant embodiments.
FIG. 10 is a perspective view of a fifth example support system and frame for use with disclosed implant embodiments.
FIG. 11 is a cross section drawing of the embodiment of FIG. 10.
FIG. 12 is a perspective view of a sixth example support system and frame for use with disclosed implant embodiments.
FIG. 13 is an exploded parts view of the embodiment of FIG. 12.
FIG. 14 is a perspective view of a seventh example support system and frame for use with disclosed implant embodiments.

### DETAILED DESCRIPTION

Embodiments of the present disclosure relate generally, for example, to spinal stabilization systems, and more particularly, to implants used as spinal stabilization systems. Embodiments of the devices and methods (surgical methods are not claimed) are described below with reference to the Figures.

The following discussion omits or only briefly describes certain components, features and functionality related to medical implants, installation tools, and associated surgical techniques, which are apparent to those of ordinary skill in the art. It is noted that various embodiments are described in detail with reference to the drawings, in which like reference numerals represent like parts and assemblies throughout the several views, where possible. Reference to various embodiments does not limit the scope of the claims appended hereto because the embodiments are examples of the inventive concepts described herein. Additionally, any example(s) set forth in this specification are intended to be non-limiting and set forth some of the many possible embodiments applicable to the appended claims. Further, particular features described herein can be used in combination with other described features in each of the various possible combinations and permutations unless the context or other statements clearly indicate otherwise.

Terms such as "same," "equal," "planar," "coplanar," "parallel," "perpendicular," etc. as used herein are intended to encompass a meaning of exactly the same while also including variations that may occur, for example, due to manufacturing processes. The term "substantially" may be used herein to emphasize this meaning, particularly when the described embodiment has the same or nearly the same functionality or characteristic, unless the context or other statements clearly indicate otherwise.

Various embodiments and components may be coated with a ceramic, titanium, and/or other biocompatible material to provide surface texturing at (a) the macro scale, (b) the micro scale, and/or (c) the nano scale, for example. Similarly, components may undergo a subtractive manufacturing process providing for surface texturing configured to facilitate osseointegration and cellular attachment and osteoblast maturation. Example surface texturing of additive and subtractive manufacturing processes may comprise (a) macro-scale structural features having a maximum peak-to-valley height of about 40 microns to about 500 microns, (b) micro-scale structural features having a maximum peak-to-valley height of about 2 microns to about 40 microns, and/or (c) nano-scale structural features having a maximum peak-to-valley height of about 0.05 microns to about 5 microns. In various embodiments, the three types of structural features may be overlapping with one another, for example. Additionally, such surface texturing may be applied to any surface, e.g., both external exposed facing surfaces of components and internal non exposed surfaces of components. Further discussion regarding exemplary surface texturing and coatings is described in, for example, U.S. Pat. No. 11,096,796, titled "Interbody spinal implant having a roughened surface topography on one or more internal surfaces," and filed on March, 4, 2013**.** Accordingly, it shall be understood that any of the described coating and texturing processes of U.S. Pat. No. 11,096,796, may be applied to any component of the various embodiments disclosed herein, e.g., the exposed surfaces and internal surfaces of endplates. Another example technique for manufacturing an orthopedic implant having surfaces with osteoinducting roughness features including micro-scale structures and nano-scale structures is disclosed in U.S. Pat. No. 10,821,000.

Various embodiments and components of this disclosure may be fabricated from biologically acceptable materials suitable for medical applications including metals, synthetic polymers, ceramics and bone material and/or their composites. For example, endcaps and/or endplates of various embodiments disclosed herein may be fabricated from materials such as stainless steel alloys, commercially pure titanium, titanium alloys, Grade 5 titanium, super-elastic titanium alloys, cobalt-chrome alloys, superelastic metallic alloys (e.g., Nitinol, super elasto-plastic metals, such as GUM METAL^{®}), ceramics and composites thereof such as calcium phosphate (e.g., SKELITE^{™}). In various embodiments, a body portion of disclosed implants may be formed of and/or include thermoplastics such as polyaryletherketone (PAEK) including polyetheretherketone (PEEK), polyetherketoneketone (PEKK) and polyetherketone (PEK), carbon-PEEK composites, PEEK-BaSO4 polymeric rubbers, polyethylene terephthalate (PET). Other components of disclosed implants may be formed of fabric, silicone, polyurethane, silicone-polyurethane copolymers, polymeric rubbers, polyolefin rubbers, hydrogels, semirigid and rigid materials, elastomers, rubbers, thermoplastic elastomers, thermoset elastomers, elastomeric composites, rigid polymers including polyphenylene, polyamide, polyimide, polyetherimide, polyethylene, epoxy, bone material including autograft, allograft, xenograft or transgenic cortical and/or corticocancellous bone, and tissue growth or differentiation factors, partially resorbable materials, such as, for example, composites of metals and calcium-based ceramics, composites of PEEK and calcium based ceramics, composites of PEEK with resorbable polymers, totally resorbable materials, such as, for example, calcium based ceramics such as calcium phosphate, tri-calcium phosphate (TCP), hydroxyapatite (HA)-TCP, calcium sulfate, or other resorbable polymers such as polyaetide, polyglycolide, polytyrosine carbonate, polycaroplaetohe, polylactic acid or polylactide and their combinations.

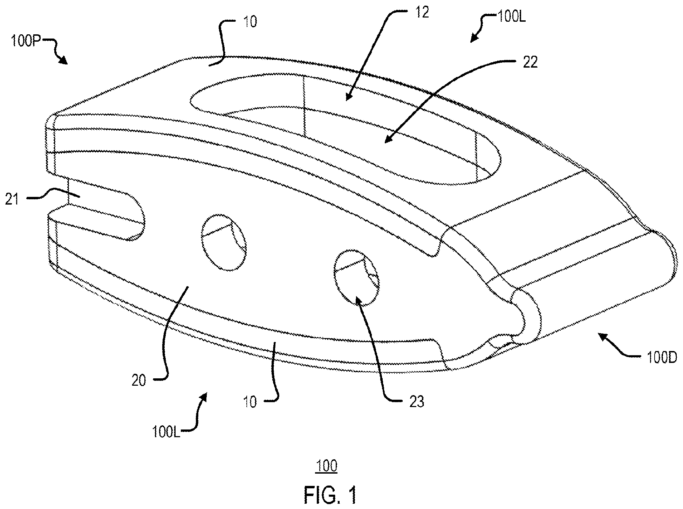

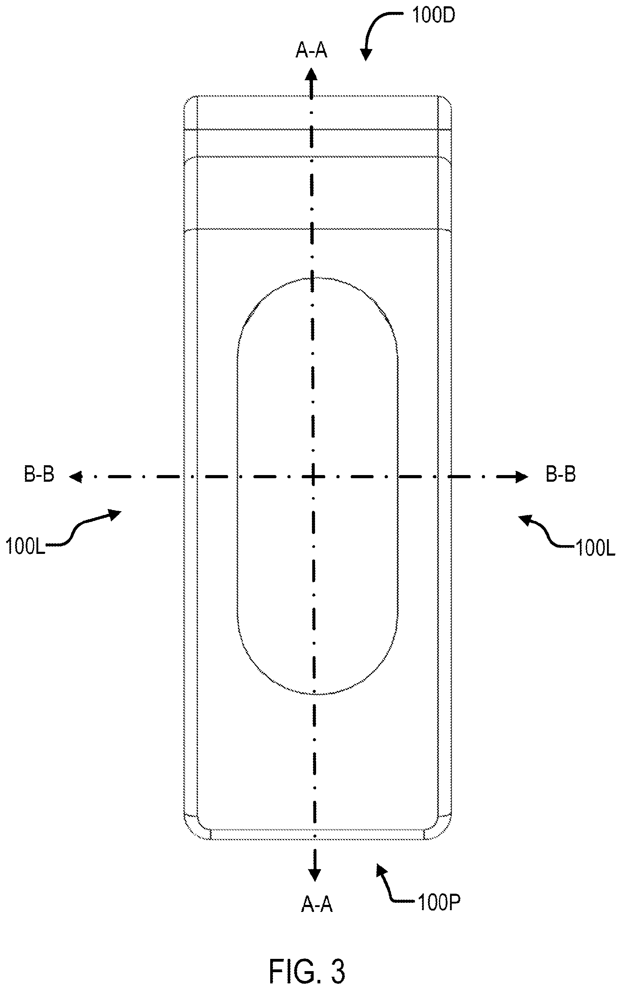

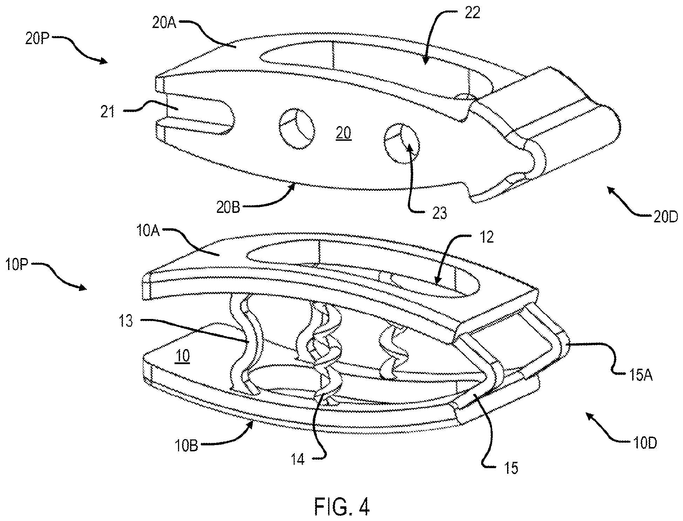

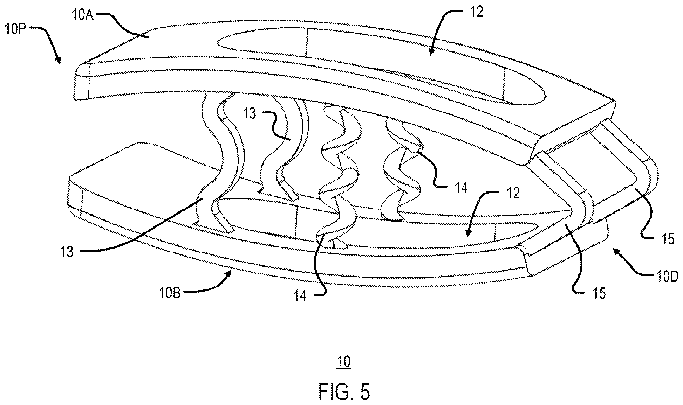

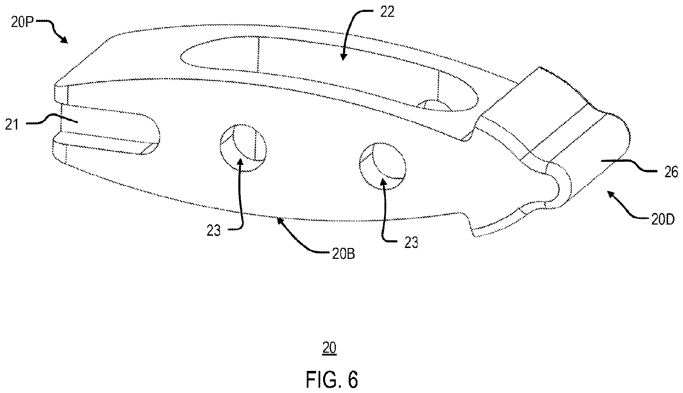

Referring generally to FIGS. 1-6 various views of an interbody implant 100 are disclosed. FIGS. 1-2 illustrate front and rear perspective views of implant 100 and FIG. 3 illustrates a top-down view of implant 100. FIG. 4 is an exploded parts view of implant 100; FIG. 5 is a perspective view of end plates of interbody implant 100; and FIG. 6 is a perspective view of a polymer body of interbody implant 100. In the example embodiment, implant 100 may be formed of an interconnected frame 10 or interconnected endplates 10 and an overmold polymer body 20 disposed between the interconnected endcaps. In some embodiments, cage 10 may function similarly to a skeleton and the body 20 to be molded within by, for example, an injection molding process. During the molding process, liquid polymer may flow within a cavity formed by the interconnected endplates to thereby surround a support structure associated with the interconnected endplates, as will be explained in further detail below.

With reference to FIG. 3, implant 100 may extend in a proximal to distal direction along longitudinal axis A-A from a proximal end 100P to a distal end 100D. Additionally, implant 100 may extend in a widthwise direction along lateral axis B-B between a first lateral end 100L and a second lateral end 100L. In various embodiments, the cross-section geometry of interbody implant 100 may have various configurations and/or shapes, such as, for example, cylindrical, round, oval, oblong, triangular, polygonal having planar or arcuate side portions, irregular, uniform, non-uniform, consistent, variable, horseshoe shape, U-shape or kidney bean shape.

As seen best in FIGS. 1-2, frame 10 includes a first opening 12 and body 20 includes a second opening 22 aligning with and adjoining first opening 12. The openings 12, 22 are configured to receive an agent, which may include bone graft (not shown) and/or other materials, as described herein, for employment in a fixation or fusion treatment, as described herein. Similarly, body 20 may include lateral openings 23 which may communicate between lateral ends 100L and openings 12, 22, for example. In one embodiment, an agent may include therapeutic polynucleotides or polypeptides and bone growth promoting material, which can be packed or otherwise disposed on or about the surfaces of the components of spinal implant 100, including inside of openings 12, 22, and/or 23. In this embodiment, lateral openings 23 adjoin with and/or communicate with openings 12, 22 to facilitate a fusion process and may be packed and/or injected with bone growth promoting material before or after insertion of implant 100.

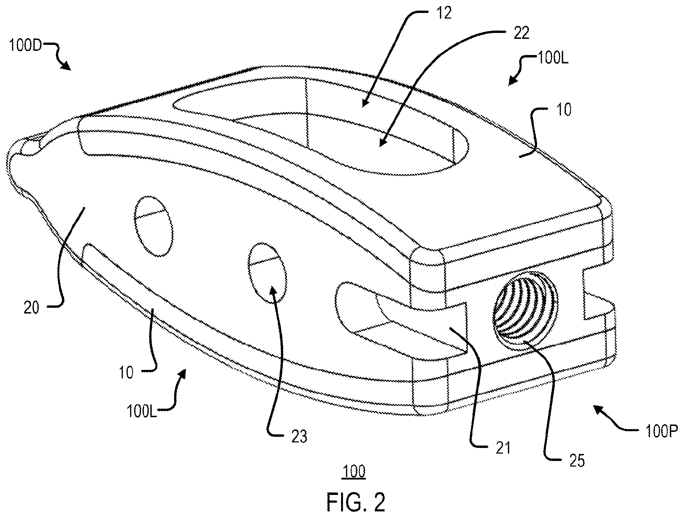

As seen best in FIG. 2, implant 100 may include various contours and/or features to facilitate insertion of implant 100 inside of a patient. In the example embodiment, a proximal end 100P of implant 100 includes gripping indentations 21 and a threaded aperture 25. In use, a surgeon may use a surgical tool to grasp onto implant 100 at gripping indentations 21 and/or by threadingly engaging a surgical tool (not illustrated) to the threaded aperture 25, for example.

Referring to FIGS 4-5, frame 10 may be interconnected by one or more compressible struts, e.g., first strut 13, second strut 14, and/or third strut 15. As illustrated, struts 13, 14, and 15 extend between an interior surface of first endplate 10A (superior endplate 10A) and an interior surface of second endplate 10B (inferior endplate 10B). First strut 13 may resemble a wave like column and/or an undulating column, second strut 14 may resemble a helical spring and/or helical column, and third strut 15 may resemble a torsion spring and/or a bent column. In various embodiments, struts 13, 14, 15 may be configured to maintain a position of the endplates 10A, 10B to form a cavity for polymer to flow within. In one aspect, struts 13, 14, 15 may be configured to have a compressible stiffness or rigidity that is less than a compressible stiffness or rigidity of the cured polymer disposed between endplates 10A, 10B. Specifically, struts 13, 14, 15 may include flexible or compressible features, such as helical or undulating shapes to enable the struts 13, 14, 15 to deflect as necessary upon application of a compressive force on the implant 100. Upon application of a compressive force, a distance between an outermost surface of first endplate 10A and an outermost surface of second endplate 20A may be reduced relative to a neutral position in which struts 13, 14, and 15 are at rest..

Referring to the embodiment of FIG. 5, frame 10 may extend in a longitudinal direction from a proximal end 10P to a distal end 10D and extend in a vertical direction between a first endplate 10A and a second endplate 10B, for example. Additionally, the first endplate 10A may be interconnected to a second endplate 10B by a structural support system that allows for some compression and/or deformity in the vertical direction, such as for example, by at least one strut 13, 14, 15. Each strut 13, 14, 15 may be disposed in any suitable location to support a position of endplates 10A, 10B for molding, and have a stiffness or rigidity that is less than a stiffness or rigidity of the cured polymer body 20. Those with skill in the art will readily understand that the stiffness of any particular strut may be determined by the type of material, the shape, design, and geometry of the strut, the length of the strut, and of course the cross-sectional thickness of the strut, among other things, for example. The disclosure herein provides numerous example frames 10, 30, 40, 50, 60, 70, 80 all having varying types, sizes, number, and locations of structural supports.

In the example embodiment of FIG. 5, a pair of curved column struts 13 are disposed on opposite lateral sides of frame 10, and a pair of helical column struts 14 are disposed on opposite lateral sides of frame 10. Additionally, a pair of torsion struts 15 having at least one bent portion 15A are disposed adject the distal end 10D. Frame 10 may be formed by a cast and mold process, an additive manufacturing process, and/or a subtractive manufacturing process. In at least one embodiment, frame 10 is formed by a metal injection molding (MIM) process such that the first endplate 10A, second endplate 10B, and the various structural supports and struts 13, 14, 15 are formed as a monolithic component.

Referring to the embodiment of FIG. 6, a cured polymeric body 20 may extend in a longitudinal direction from a proximal end 20P to a distal end 20D. Body 20 may include a superior bearing surface 20A that directly contacts a corresponding interior surface of superior endplate 10A, for example. Similarly, body 20 may include an inferior bearing surface 20B that directly contacts a corresponding interior surface of inferior endplate 10B, for example. In this embodiment, body 20 may include angled nose portion 26 at the distal end 20D. In various embodiments, body 20 may be formed of any suitable biocompatible polymer and various combinations and/or layers of biocompatible polymers. In at least one embodiment, body 20 is formed by a mold in place process. For example, once frame 10 is formed, frame 10 may serve as a mold and/or function as a frame in which body 20 may be molded too. In another embodiment, frame 10 may serve as an injection mold defining a cavity for receiving flowable polymeric material. In addition to frame 10, other structures such as cutouts, inserts, formwork, temporary walls, runners, gates, ribs, bosses, etc. may be utilized to ensure that body 20 takes a specific shape corresponding to the size and shape of frame 10. In the example embodiment, body 20 is formed of a polymeric material that completely surrounds the struts 13, 14, 15 and abuts and bonds directly against interior surfaces of endplates 10A, 10B. In some embodiments, frame 10 may be placed inside of an outer mold having a suitable size and shape for placing frame 10 inside of. Once frame 10 is placed inside of outer mold, the endplates 10A, 10B are held in position by one or more struts 13-15, and a homogenous polymeric material may be injected inside of outer mold such that the material is allowed to surround struts, abut endplates, and cure. In some embodiments, an outer mold, along with endplates 10A, 10B, may approximate the final shape of implant 100. More specifically, body 20 may have a shape defined by endplates 10A, 10B and a distal end 20D of body 20 may have a shape defined by the outer mold (e.g., angled nose portion 26). Additionally, in some aspects, a subtractive manufacturing process may be applied to the polymeric material of body 20 until it has a specific desired shape. For example, a subtractive manufacturing process may be used to remove additional polymeric material at distal end 20D until the smooth shaped nose portion 26 is formed. Similarly, polymeric material may be removed from body 20 to form gripping indentation 21, openings 22, 23, and to expose the outer surfaces of the first endplate 10A and second endplate 10B of frame 10. Thereafter, another subtractive manufacturing process may be applied to the outer surfaces of the metallic structure of frame 10 to create various surface texturing patterns that promote osteointegration. In some embodiments, body 20 may comprise a honeycomb structure.

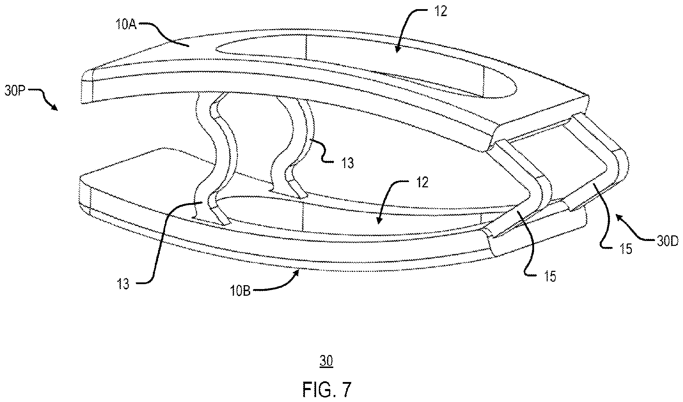

FIG. 7 is a perspective view of a second example support system and frame 30. Frame 30 may have the same, similar, and/or substantially the same features and functionality as explained above with respect to frame 10. For example, frame 30 may function as a skeleton with which body 20 may be formed inside of and/or around. In this embodiment, frame 30 includes a pair of curved column struts 13 adjacent a medial portion of implant 100. Additionally, frame 30 includes a pair of torsion struts 15 adjacent a distal end 30D of frame 30 opposite proximal end 30P. In this embodiment, a combined compressive stiffness strength of frame 30 may be less than that of frame 10 on account of frame 10 having two additional helical struts 14 in the medial portion of frame 10.

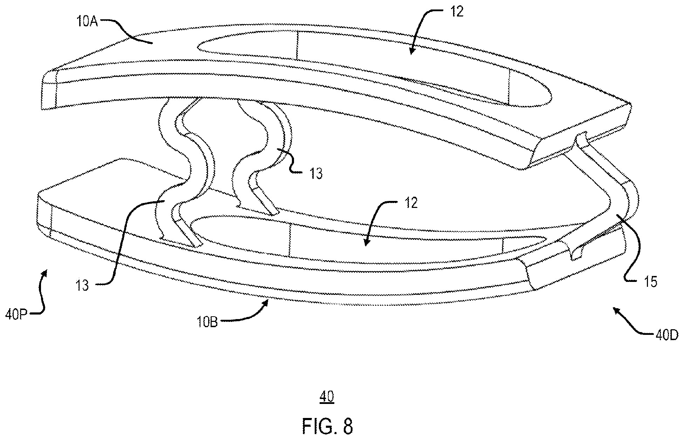

FIG. 8 is a perspective view of a third example support system and frame 40. Frame 40 may have the same, similar, and/or substantially the same features and functionality as explained above with respect to frames 10, and 30. For example, frame 40 may function as a framework with which body 20 may be formed inside of and/or around. In this embodiment, frame 40 includes a pair of curved column struts 13 adjacent a medial portion of implant 100. Additionally, frame 40 includes a single torsion strut 15 that is centered on a distal end 40D of frame 40 opposite proximal end 40P. This configuration may be advantageous in that it may allow the distal end to compress further (more easily and/or less force) than frame 30 on account of having a single torsion strut 15. Additionally, this configuration may allow for further lateral bending at the left and right proximal edges on account of the torsion strut 15 being centered. In this embodiment, a combined compressive stiffness strength of frame 40 may be less than that of frame 30 on account of frame 30 having an additional torsion strut 15 at the distal end 30D.

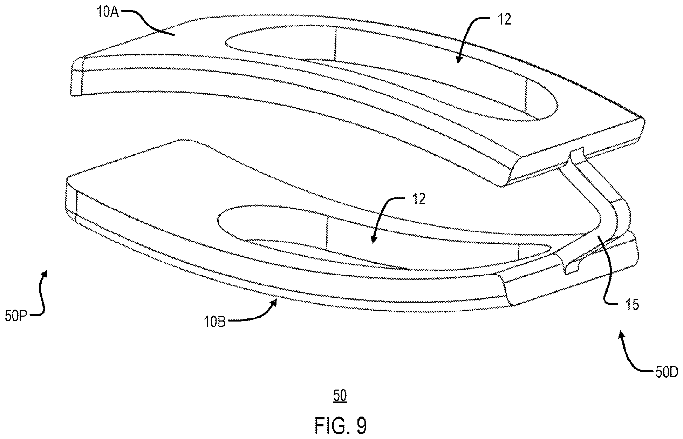

FIG. 9 is a perspective view of a fourth example support system and frame 50. Frame 50 may have the same, similar, and/or substantially the same features and functionality as explained above with respect to frames 10, 30, and 40. For example, frame 50 may function as a frame with which body 20 may be formed inside of and/or around. In this embodiment, frame 50 includes a single torsion strut 15 that is centered on a distal end 50D of frame 50. In this embodiment, a combined compressive stiffness strength of frame 50 may be less than that of frame 40 on account of frame 40 having an additional pair of struts 13.

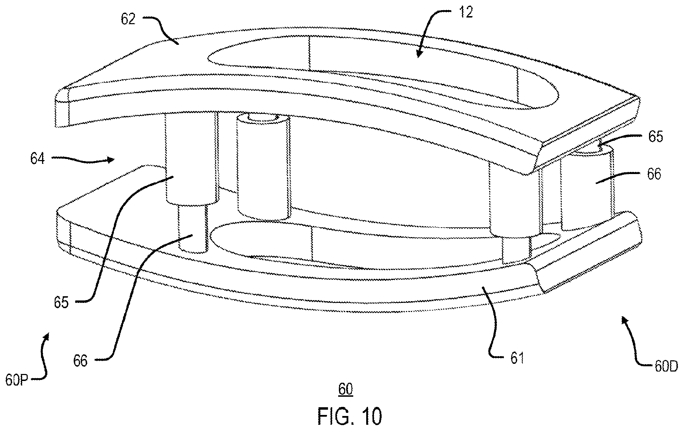

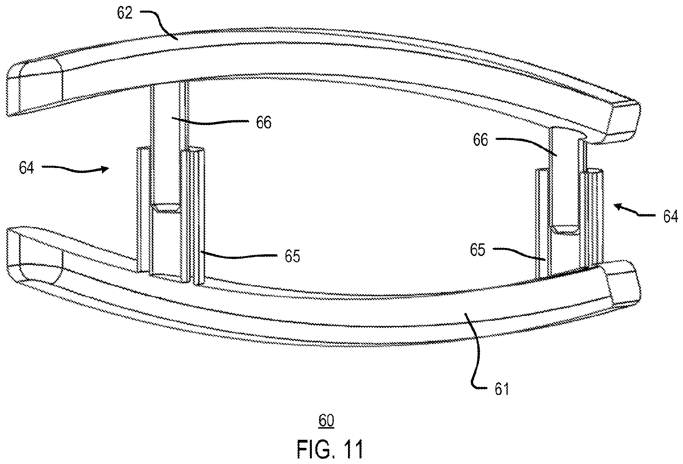

FIG. 10 is a perspective view of a fifth example support system and frame 60 and FIG. 11 is a cross section view of frame 60. Frame 60 may have the same, similar, and/or substantially the same features and functionality as explained above with respect to frames 10, 30, 40, and 50. For example, frame 60 may function as a frame with which body 20 may be formed inside of and/or around. In this embodiment, frame 60 includes a structural support system including a plurality of translating struts in the form of pistons 64. In this embodiment, pistons 64 may interconnect the interior of superior endplate 62 with the interior of inferior endplate 61. Additionally, a first pair of pistons 64 may be disposed adjacent a distal end 60D and a second pair of pistons 64 may be disposed adjacent a proximal end 60P. At least one advantage of pistons 64 may be that they allow for a relative height of the frame 60 to be variable or adjustable. For example, pistons 64 may allow for a relative distance between an outermost surface of a superior endplate 62 and an inferior endplate 61 to be adjusted, as desired, prior to injection molding of body 20. This configuration allows a manufacturer to establish a patient specific and appropriate height of frame 60, and after establishing this height the endplates 61, 62 may be positioned at the appropriate height via pistons 64, and body 20 may be molded to frame 60 as explained above. In some embodiments, polymeric material during the molding process may surround and/or encapsulate the entirety of pistons 64, and in this embodiment the compressive stiffness of implant 100 would correspond to a compressive stiffness of body 20 due to pistons 64 not providing additional structural stiffness.

As seen best in the cross section drawing of FIG. 11, pistons 64 may include a pin 66 that is disposed inside of a hollow cylinder 65. In one aspect, the positions of endplates 61, 62 is maintained by a friction fit between pin 66 and cylinder 65. In other aspects, hollow cylinder 65 may be a void space or may be filled with an additional biocompatible material, e.g., silicon.

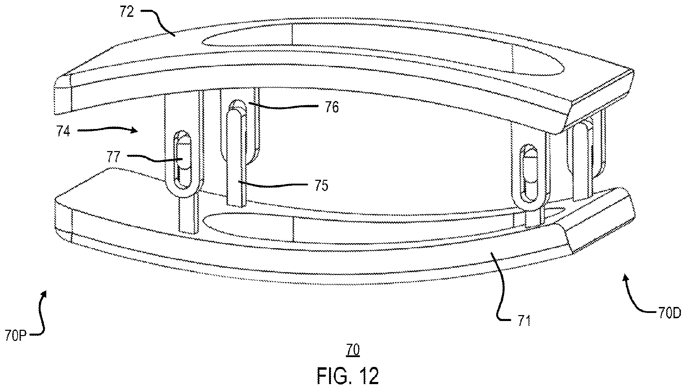

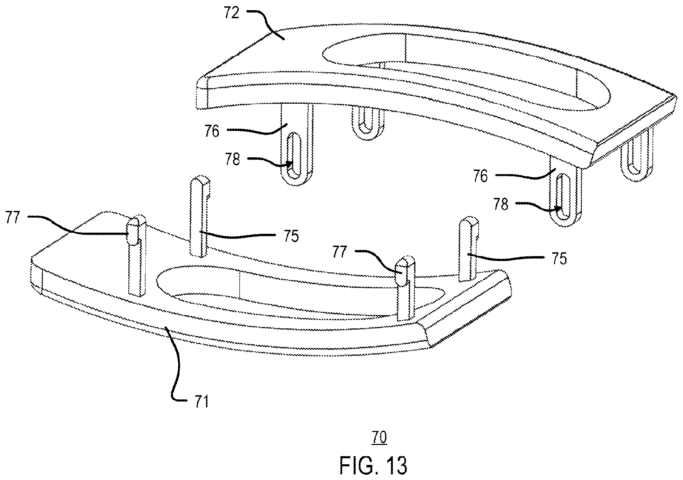

FIG. 12 is a perspective view of a sixth example support system and frame 70 and FIG. 13 is an exploded parts view of frame 70. Frame 70 may have the same, similar, and/or substantially the same features and functionality as explained above with respect to frames 10, 30, 40, 50, and 60. For example, frame 70 may function as a frame with which body 20 may be formed inside of and/or around. In this embodiment, frame 70 includes a structural support system including a plurality of translating struts in the form of linkage assemblies 74. In this embodiment, each linkage 74 may interconnect the interior of superior endplate 72 with the interior of inferior endplate 71. Additionally, a first pair of linkages 74 may be disposed adjacent a distal end 70D and a second pair of linkages 74 may be disposed adjacent a proximal end 70P. At least one advantage of linkages 74 may be that they allow for a relative height of the frame 70 to be variable or adjustable. For example, linkages 74 may allow for a relative distance between an outermost surface of a superior endplate 72 and an inferior endplate 71 to be adjusted, as desired, prior to injection molding of body 20. This configuration allows a manufacturer to establish a patient specific and appropriate height of frame 70, and after establishing this height the endplates 71, 72 may be positioned at the appropriate height via linkages 74, and body 20 may be molded to frame 70 as explained above. In some embodiments, polymeric material during the molding process may surround and/or encapsulate the entirety of linkages 74, and in this embodiment the total compressive stiffness of implant 100 would correspond to a compressive stiffness of body 20 due to linkages 74 not providing additional structural stiffness.

As seen best in the exploded view drawing of FIG. 13, linkages 74 may include a first post 76 having a vertical slot 78 and a second post 75 having a lateral protrusion 77. Once assembled, the lateral protrusions 77 may extend laterally through vertical slot 78 thereby allowing some relative motion in the vertical direction between the superior endplate 72 and inferior endplate 71. In one aspect, the positions of endplates 71, 72 is maintained by a friction fit between protrusion 71 and the slot 78. In other aspects, vertical slot 78 may be a void space or may be filled with an additional biocompatible material, e.g., silicon. Additionally, a patient specific height of frame 70 and/or a lordotic / kyphotic angle of inclination may be set by positioning lateral protrusions 77 within slots 78 as desired and/or determined during preoperative planning. For example, a first monolithic component comprising the superior endplate 72 and posts 76 with slots 78 may be formed and a second monolithic component comprising the inferior endplate 71 and posts 75 with lateral protrusions 77 may be formed. Thereafter, a manufacturer may position the superior and inferior endplates 72, 71 as desired and form the body 20 by a molding process as explained previously.

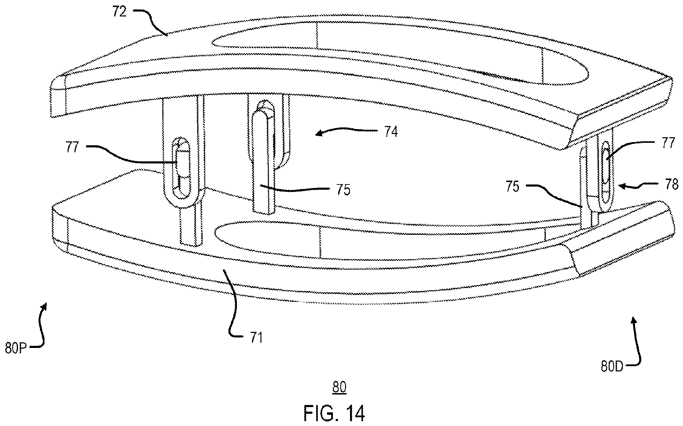

FIG. 14 is a perspective view of a seventh example support system and frame 80 for use with disclosed implant embodiments. Frame 80 may have the same, similar, and/or substantially the same features and functionality as explained above with respect to frames 10, 30, 40, 50, 60, and 70. For example, frame 80 may function as a frame with which body 20 may be formed inside of and/or around. In this embodiment, frame 80 has a similar structural system as explained previously with respect to frame 70. Accordingly, duplicative explanation will be omitted. In this embodiment, a single translating strut may be disposed on a distal end 80D and a pair of translating struts 74 may be disposed in a medial portion and/or adjacent a proximal end 80P.

In various embodiments, attributes of frames 10, 30, 40, 50, 60, 70, and 80 may be mix and matched for one another unless the context clearly dictates that such features and components are mutually exclusive. Additionally, any of frames 10, 30, 40, 50, 60, 70 and 80 may be formed of a biocompatible metallic material such as titanium and body 20 may be formed of any polymer material or polymeric material layers. In some embodiments, body 20 may be formed as a honeycomb structure which may assist in obtaining a body 20 having a desired flexibility and compressive stiffness. In various embodiments, any of the aforementioned components herein can be manufactured, fabricated or produced via machining, molding, casting, sintering, and/or additive manufacturing such as 3D-printing or laser sintering.

As a general principle of this disclosure, it should be understood that in some embodiments a first localized compressive stiffness may be greater than or less than a second localized compressive stiffness. For example, in some embodiments a first localized compressive stiffness of a leading end (distal end) may be less than that of a proximal end. Additionally, it should be understood that a total compressive stiffness of implant 100 may be the sum of the stiffness of the interconnected structural supports of cages 10, 30, 40, 50, 60, 70, and 80 and the stiffness of the body 20. In some embodiments, care may be taken such that a total stiffness of the implant 100 is well suited for the particular region of interest. For example, when manufacturing a relatively Large Lumbar Interbody Device (Laterally inserted device) an example stiffness may be about 33,500 N/mm. When manufacturing a relatively strong and Small Lumbar Interbody Device (Posterior inserted device) an example stiffness may be about 22,220 N/mm. In other embodiments calling for a relatively weaker Small Lumbar Interbody Device (Posteriorly inserted device) an example stiffness may be about 12,500 N/mm. When manufacturing a Cervical Interbody Device an example stiffness may be about 11,500 N/mm. Additionally, it shall be understood that the term "about" encompasses a variation of at least +/- 10% from the example values provide herein.

As another general principle of this disclosure, it should be understood that the relative stiffness of the interconnected structural supports of frames 10, 30, 40, 50, 60, 70, and 80 is less than the stiffness of the body 20. Accordingly, in some embodiments a first stiffness of the interconnected structural supports of frame (10, 30, 40, 50, 60, 70, and 80 may be about 20% to about 80% of a second stiffness of the body 20 by itself. In one preferred embodiment, a first stiffness of the interconnected structural supports of frame may be about 50% of the stiffness of the body 20 by itself.

It should be understood that various aspects disclosed herein may be combined in different combinations than the combinations specifically presented in the description and accompanying drawings. For example, features, functionality, and components from one embodiment may be combined with another embodiment and vice versa unless the context clearly indicates otherwise. Similarly, features, functionality, and components may be omitted unless the context clearly indicates otherwise. It should also be understood that, depending on the example, certain acts or events of any of the processes or methods (surgical methods are not claimed) described herein may be performed in a different sequence, may be added, merged, or left out altogether (e.g., all described acts or events may not be necessary to carry out the techniques).

Unless otherwise specifically defined herein, all terms are to be given their broadest possible interpretation including meanings implied from the specification as well as meanings understood by those skilled in the art and/or as defined in dictionaries, treatises, etc. It must also be noted that, as used in the specification and the appended claims, the singular forms "a," "an" and "the" include plural referents unless otherwise specified, and that the terms "comprises" and/ or "comprising," when used in this specification, specify the presence of stated features, elements, and/or components, but do not preclude the presence or addition of one or more other features, steps, operations, elements, components, and/or groups thereof.

## Claims

1. An interbody implant (100), comprising:
a metallic frame (10, 30, 40, 50, 60, 70, 80) including an interconnected superior endplate (10A, 62, 72) and inferior endplate (10B, 61, 71), the metallic frame (10, 30, 40, 50, 60, 70, 80) having a first compressive stiffness; and
a polymeric body (20) formed to the metallic frame (10, 30, 40, 50, 60, 70, 80) by an overmold process, the polymeric body (20) having a second compressive stiffness,
wherein the first compressive stiffness of the metallic frame (10, 30, 40, 50, 60, 70, 80) is about 20% to about 80% of the second stiffness of the polymeric body (20),
wherein the metallic frame (10, 30, 40, 50, 60, 70, 80) includes a first opening (12), and the polymeric body (20) includes a second opening (22) aligning with and adjoining the first opening (12),
wherein the first opening (12) and the second opening (22) are configured to receive an agent for employment in a fixation or fusion treatment, and,
wherein the interbody implant (100) further comprises at least one compressible strut (13, 14, 15) interconnecting the superior endplate (10A, 62, 72) and inferior endplate (10B, 61, 71), the at least one compressible strut (13, 14, 15) defining the first compressive stiffness.

2. The interbody implant (100) of claim 1, wherein the at least one compressible strut (13, 14, 15) is chosen from the group comprising: an undulating column strut (13), a helical column strut (14), and a torsion strut (15).

3. The interbody implant (100) of claim 1, wherein the at least one compressible strut is a torsion strut (15), wherein the torsion strut (15) is disposed adjacent a distal end (10D, 30D, 40D, 50D, 60D, 70D, 80D) of the metallic frame (10, 30, 40, 50, 60, 70, 80).

4. The interbody implant (100) of claim 1, further comprising a plurality of compressible struts (13, 14, 15), each strut of the plurality of compressible struts (13, 14, 15) being chosen from the group comprising: undulating column struts (13), helical column struts (14), and torsion struts (15), wherein optionally each strut (13, 14, 15) of the plurality of compressible struts (13, 14, 15) is a same type of strut (13, 14, 15), or wherein optionally at least two struts (13, 14, 15) of the plurality of compressible struts (13, 14, 15) are a different type of strut (13, 14, 15).

5. The interbody implant (100) of claim 4, wherein the first compressive stiffness of the metallic frame (10, 30, 40, 50, 60, 70, 80) is about 50% of the second stiffness of the polymeric body (20).

6. The interbody implant (100) of claim 1, wherein exposed surfaces of the superior endplate (10A, 62, 72) and inferior endplate (10B, 61, 71) comprise roughened and/or porous surfaces configured to facilitate bone growth to the superior endplate (10A, 62, 72) and inferior endplate (10B, 61, 71).

7. The interbody implant (100) of claim 1, wherein the polymeric body (20) comprises a homogenous material.

8. The interbody implant (100) of claim 1, wherein a sum of the first compressive stiffness of the metallic frame (10, 30, 40, 50, 60, 70, 80) and the second stiffness of the polymeric body (20) is about 33,500 N/mm to about 11,500 N/mm.

9. An interbody implant (100) formed at least partially by an overmold process, comprising:
a metallic frame (10, 30, 40, 50, 60, 70, 80) including a superior endplate (10A, 62, 72) and an inferior endplate (10B, 61, 71) interconnected by a plurality of translating struts, the metallic frame (10, 30, 40, 50, 60, 70, 80) having a first compressive stiffness defined by the at least one translating strut; and
a polymeric body (20) formed to the metallic frame (10, 30, 40, 50, 60, 70, 80) by an overmold process, the polymeric body (20) having a second compressive stiffness,
wherein the first compressive stiffness of the metallic frame (10, 30, 40, 50, 60, 70, 80) is about 20% to about 80% of the second stiffness of the polymeric body (20),
wherein the metallic frame (10, 30, 40, 50, 60, 70, 80) includes a first opening (12), and the polymeric body (20) includes a second opening (22) aligning with and adjoining the first opening (12), and
wherein the first opening (12) and the second opening (22) are configured to receive an agent for employment in a fixation or fusion treatment.

10. The interbody implant (100) of claim 9, wherein the plurality of translating struts comprise pistons (64).

11. The interbody implant (100) of claim 10, wherein each piston comprises a pin and a hollow cylinder (65).

12. The interbody implant (100) of claim 9, wherein the plurality of translating struts comprise linkage assemblies (74).

13. The interbody implant (100) of claim 12, wherein each linkage assembly (74) comprises a first post (76) with a slot (78) and a second post (75) with a lateral protrusion (77).

14. The interbody implant (100) of claim 13, wherein each lateral protrusion (77) is disposed in a respective slot (78) via a friction fit.

15. The interbody implant (100) of claim 9, wherein a sum of the first compressive stiffness of the metallic frame (10, 30, 40, 50, 60, 70, 80) and the second stiffness of the polymeric body (20) is about 33,500 N/mm to about 11,500 N/mm.

## Patentansprüche

1. Zwischenkörperimplantat (100), umfassend:
einen metallischen Rahmen (10, 30, 40, 50, 60, 70, 80), der eine miteinander verbundene obere Endplatte (10A, 62, 72) und untere Endplatte (10B, 61, 71) einschließt, wobei der metallische Rahmen (10, 30, 40, 50, 60, 70, 80) eine erste Drucksteifigkeit aufweist; und
einen polymeren Körper (20), der durch einen Umspritzungsprozess an den metallischen Rahmen (10, 30, 40, 50, 60, 70, 80) ausgebildet ist, wobei der polymere Körper (20) eine zweite Drucksteifigkeit aufweist,
wobei die erste Drucksteifigkeit des metallischen Rahmens (10, 30, 40, 50, 60, 70, 80) etwa 20 % bis etwa 80 % der zweiten Steifigkeit des polymeren Körpers (20) beträgt,
wobei der metallische Rahmen (10, 30, 40, 50, 60, 70, 80) eine erste Öffnung (12) einschließt und der polymere Körper (20) eine zweite Öffnung (22) einschließt, die an der ersten Öffnung (12) ausgerichtet ist und an diese angrenzt,
wobei die erste Öffnung (12) und die zweite Öffnung (22) konfiguriert sind, um ein Mittel für einen Gebrauch bei einer Fixierungs- oder Fusionsbehandlung aufzunehmen, und,
wobei das Zwischenkörperimplantat (100) ferner mindestens eine komprimierbare Strebe (13, 14, 15) umfasst, die die obere Endplatte (10A, 62, 72) und die untere Endplatte (10B, 61, 71) miteinander verbindet, wobei die mindestens eine komprimierbare Strebe (13, 14, 15) die erste Drucksteifigkeit definiert.

2. Zwischenkörperimplantat (100) nach Anspruch 1, wobei die mindestens eine komprimierbare Strebe (13, 14, 15) aus der Gruppe ausgewählt ist umfassend: eine wellenförmige Säulenstrebe (13), eine schraubenförmige Säulenstrebe (14) und eine Torsionsstrebe (15).

3. Zwischenkörperimplantat (100) nach Anspruch 1, wobei die mindestens eine komprimierbare Strebe eine Torsionsstrebe (15) ist, wobei die Torsionsstrebe (15) angrenzend an ein distales Ende (10D, 30D, 40D, 50D, 60D, 70D, 80D) des metallischen Rahmens (10, 30, 40, 50, 60, 70, 80) eingerichtet ist.

4. Zwischenkörperimplantat (100) nach Anspruch 1, ferner umfassend eine Vielzahl von komprimierbaren Streben (13, 14, 15), wobei jede Strebe der Vielzahl von komprimierbaren Streben (13, 14, 15) aus der Gruppe ausgewählt ist, umfassend: wellenförmige Säulenstreben (13), schraubenförmige Säulenstreben (14) und Torsionsstreben (15), wobei optional jede Strebe (13, 14, 15) der Vielzahl von komprimierbaren Streben (13, 14, 15) eine gleiche Art von Strebe (13, 14, 15) ist, oder wobei optional mindestens zwei Streben (13, 14, 15) der Vielzahl von komprimierbaren Streben (13, 14, 15) eine unterschiedliche Art von Strebe (13, 14, 15) sind.

5. Zwischenkörperimplantat (100) nach Anspruch 4, wobei die erste Drucksteifigkeit des metallischen Rahmens (10, 30, 40, 50, 60, 70, 80) etwa 50 % der zweiten Steifigkeit des polymeren Körpers (20) beträgt.

6. Zwischenkörperimplantat (100) nach Anspruch 1, wobei freiliegende Oberflächen der oberen Endplatte (10A, 62, 72) und unteren Endplatte (10B, 61, 71) aufgeraute und/oder poröse Oberflächen umfassen, die konfiguriert sind, um Knochenwachstum an der oberen Endplatte (10A, 62, 72) und unteren Endplatte (10B, 61, 71) zu erleichtern.

7. Zwischenkörperimplantat (100) nach Anspruch 1, wobei der polymere Körper (20) ein homogenes Material umfasst.

8. Zwischenkörperimplantat (100) nach Anspruch 1, wobei eine Summe der ersten Drucksteifigkeit des metallischen Rahmens (10, 30, 40, 50, 60, 70, 80) und der zweiten Steifigkeit des polymeren Körpers (20) etwa 33.500 N/mm bis etwa 11.500 N/mm beträgt.

9. Zwischenkörperimplantat (100), das mindestens teilweise durch einen Umspritzungsprozess ausgebildet ist, umfassend:
einen metallischen Rahmen (10, 30, 40, 50, 60, 70, 80), der eine obere Endplatte (10A, 62, 72) und eine untere Endplatte (10B, 61, 71) einschließt, die durch eine Vielzahl von sich verschiebenden Streben miteinander verbunden sind, wobei der metallische Rahmen (10, 30, 40, 50, 60, 70, 80) eine erste Drucksteifigkeit aufweist, die durch die mindestens eine sich verschiebende Strebe definiert ist; und
einen polymeren Körper (20), der durch einen Umspritzungsprozess an den metallischen Rahmen (10, 30, 40, 50, 60, 70, 80) ausgebildet ist, wobei der polymere Körper (20) eine zweite Drucksteifigkeit aufweist,
wobei die erste Drucksteifigkeit des metallischen Rahmens (10, 30, 40, 50, 60, 70, 80) etwa 20 % bis etwa 80 % der zweiten Steifigkeit des polymeren Körpers (20) beträgt,
wobei der metallische Rahmen (10, 30, 40, 50, 60, 70, 80) eine erste Öffnung (12) einschließt, und der polymere Körper (20) eine zweite Öffnung (22) einschließt, die an der ersten Öffnung (12) ausgerichtet ist und an diese angrenzt, und
wobei die erste Öffnung (12) und die zweite Öffnung (22) konfiguriert sind, um ein Mittel für den Gebrauch bei einer Fixierungs- oder Fusionsbehandlung aufzunehmen.

10. Zwischenkörperimplantat (100) nach Anspruch 9, wobei die Vielzahl von sich verschiebenden Streben Kolben (64) umfasst.

11. Zwischenkörperimplantat (100) nach Anspruch 10, wobei jeder Kolben einen Stift und einen hohlen Zylinder (65) umfasst.

12. Zwischenkörperimplantat (100) nach Anspruch 9, wobei die Vielzahl von sich verschiebenden Streben Verknüpfungsanordnungen (74) umfasst.

13. Zwischenkörperimplantat (100) nach Anspruch 12, wobei jede Verknüpfungsanordnung (74) einen ersten Pfosten (76) mit einem Schlitz (78) und einen zweiten Pfosten (75) mit einem seitlichen Vorsprung (77) umfasst.

14. Zwischenkörperimplantat (100) nach Anspruch 13, wobei jeder laterale Vorsprung (77) über einen Reibschluss in einem jeweiligen Schlitz (78) eingerichtet ist.

15. Zwischenkörperimplantat (100) nach Anspruch 9, wobei eine Summe der ersten Drucksteifigkeit des metallischen Rahmens (10, 30, 40, 50, 60, 70, 80) und der zweiten Steifigkeit des polymeren Körpers (20) etwa 33.500 N/mm bis etwa 11.500 N/mm beträgt.

## Revendications

1. Implant intersomatique (100), comprenant :
un cadre métallique (10, 30, 40, 50, 60, 70, 80) comportant une plaque d'extrémité supérieure (10A, 62, 72) et une plaque d'extrémité inférieure (10B, 61, 71) interconnectées, le cadre métallique (10, 30, 40, 50, 60, 70, 80) ayant une première rigidité en compression ; et
un corps polymère (20) formé au cadre métallique (10, 30, 40, 50, 60, 70, 80) par un procédé de surmoulage, le corps polymère (20) ayant une seconde rigidité en compression,
dans lequel la première rigidité en compression du cadre métallique (10, 30, 40, 50, 60, 70, 80) est d'environ 20 % à environ 80 % de la seconde rigidité du corps polymère (20),
dans lequel le cadre métallique (10, 30, 40, 50, 60, 70, 80) comporte une première ouverture (12), et le corps polymère (20) comporte une seconde ouverture (22) s'alignant avec la première ouverture (12) et étant adjacente à celle-ci,
dans lequel la première ouverture (12) et la seconde ouverture (22) sont conçues pour recevoir un agent destiné à être employé dans un traitement de fixation ou de fusion, et,
dans lequel l'implant intersomatique (100) comprend en outre au moins une entretoise compressible (13, 14, 15) reliant la plaque d'extrémité supérieure (10A, 62, 72) et la plaque d'extrémité inférieure (10B, 61, 71), l'au moins une entretoise compressible (13, 14, 15) définissant la première rigidité en compression.

2. Implant intersomatique (100) selon la revendication 1, dans lequel l'au moins une entretoise compressible (13, 14, 15) est choisie dans le groupe comprenant : une entretoise de colonne ondulée (13), une entretoise de colonne hélicoïdale (14) et une entretoise de torsion (15).

3. Implant intersomatique (100) selon la revendication 1, dans lequel l'au moins une entretoise compressible est une entretoise de torsion (15), dans lequel l'entretoise de torsion (15) est disposée de manière adjacente à une extrémité distale (10D, 30D, 40D, 50D, 60D, 70D, 80D) du cadre métallique (10, 30, 40, 50, 60, 70, 80).

4. Implant intersomatique (100) selon la revendication 1, comprenant en outre une pluralité d'entretoises compressibles (13, 14, 15), chaque entretoise de la pluralité d'entretoises compressibles (13, 14, 15) étant choisie dans le groupe comprenant : des entretoises de colonne ondulées (13), des entretoises de colonne hélicoïdales (14) et des entretoises de torsion (15), dans lequel éventuellement chaque entretoise (13, 14, 15) de la pluralité d'entretoises compressibles (13, 14, 15) est un même type d'entretoise (13, 14, 15), ou dans lequel éventuellement au moins deux entretoises (13, 14, 15) de la pluralité d'entretoises compressibles (13, 14, 15) sont un type différent d'entretoise (13, 14, 15).

5. Implant intersomatique (100) selon la revendication 4, dans lequel la première rigidité en compression du cadre métallique (10, 30, 40, 50, 60, 70, 80) est d'environ 50 % de la seconde rigidité du corps polymère (20).

6. Implant intersomatique (100) selon la revendication 1, dans lequel les surfaces exposées de la plaque d'extrémité supérieure (10A, 62, 72) et de la plaque d'extrémité inférieure (10B, 61, 71) comprennent des surfaces rugueuses et/ou poreuses conçues pour faciliter la croissance osseuse à la plaque d'extrémité supérieure (10A, 62, 72) et à la plaque d'extrémité inférieure (10B, 61, 71).

7. Implant intersomatique (100) selon la revendication 1, dans lequel le corps polymère (20) comprend un matériau homogène.

8. Implant intersomatique (100) selon la revendication 1, dans lequel une somme de la première rigidité en compression du cadre métallique (10, 30, 40, 50, 60, 70, 80) et de la seconde rigidité du corps polymère (20) est d'environ 33 500 N/mm à environ 11 500 N/mm.

9. Implant intersomatique (100) formé au moins partiellement par un procédé de surmoulage, comprenant :
un cadre métallique (10, 30, 40, 50, 60, 70, 80) comportant une plaque d'extrémité supérieure (10A, 62, 72) et une plaque d'extrémité inférieure (10B, 61, 71) interconnectées par une pluralité d'entretoises de translation, le cadre métallique (10, 30, 40, 50, 60, 70, 80) ayant une première rigidité en compression définie par l'au moins une entretoise de translation ; et
un corps polymère (20) formé au cadre métallique (10, 30, 40, 50, 60, 70, 80) par un procédé de surmoulage, le corps polymère (20) ayant une seconde rigidité en compression,
dans lequel la première rigidité en compression du cadre métallique (10, 30, 40, 50, 60, 70, 80) est d'environ 20 % à environ 80 % de la seconde rigidité du corps polymère (20),
dans lequel le cadre métallique (10, 30, 40, 50, 60, 70, 80) comporte une première ouverture (12), et le corps polymère (20) comporte une seconde ouverture (22) s'alignant avec la première ouverture (12) et étant adjacente à celle-ci, et
dans lequel la première ouverture (12) et la seconde ouverture (22) sont conçues pour recevoir un agent destiné à être employé dans un traitement de fixation ou de fusion.

10. Implant intersomatique (100) selon la revendication 9, dans lequel la pluralité d'entretoises de translation comprend des pistons (64).

11. Implant intersomatique (100) selon la revendication 10, dans lequel chaque piston comprend une broche et un cylindre creux (65).

12. Implant intersomatique (100) selon la revendication 9, dans lequel la pluralité d'entretoises de translation comprend des ensembles de liaison (74).

13. Implant intersomatique (100) selon la revendication 12, dans lequel chaque ensemble de liaison (74) comprend un premier montant (76) avec une fente (78) et un second montant (75) avec une protubérance latérale (77).

14. Implant intersomatique (100) selon la revendication 13, dans lequel chaque protubérance latérale (77) est disposée dans une fente respective (78) par l'intermédiaire d'un ajustement par friction.

15. Implant intersomatique (100) selon la revendication 9, dans lequel une somme de la première rigidité en compression du cadre métallique (10, 30, 40, 50, 60, 70, 80) et de la seconde rigidité du corps polymère (20) est d'environ 33 500 N/mm à environ 11 500 N/mm.
